(19) Europäisches Patentamt
European Patent Office
Office européen des brevets

(11) Veröffentlichungsnummer: **0 130 519**
**A2**

(12) # EUROPÄISCHE PATENTANMELDUNG

(21) Anmeldenummer: **84107253.1**

(22) Anmeldetag: **25.06.84**

(51) Int. Cl.⁴: **C 07 D 253/06**, A 01 N 43/707
// C07C121/76, C07C49/233,
C07C53/128, C07C43/225,
C07C21/073, C07C19/02

(30) Priorität: **02.07.83 DE 3323935**

(43) Veröffentlichungstag der Anmeldung: **09.01.85**
**Patentblatt 85/2**

(84) Benannte Vertragsstaaten: **BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Kranz, Eckart, Dr., Am Acker 9,**
**D-5600 Wuppertal 1 (DE)**
Erfinder: **Jautelat, Manfred, Dr., Muellersbaum 28,**
**D-5093 Burscheid 1 (DE)**
Erfinder: **Eue, Ludwig, Dr., Paul-Klee-Strasse 36,**
**D-5090 Leverkusen 1 (DE)**
Erfinder: **Schmidt, Robert R., Dr., Im Waldwinkel 110,**
**D-5060 Bergisch Gladbach 2 (DE)**

(54) **6-(2,3-Dimethyl-but-2-yl)-1,2,4-triazin-5-on-Derivate.**

(57) Neue 6-(2,3-Dimethyl-but-2-yl)-1,2,4-triazin-5-on-Derivate der allgemeinen Formel (I)

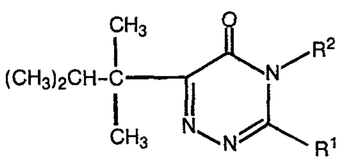

(I)

in welcher
$R^1$ für Alkylthio, Alkylamino oder Dialkylamino steht und
$R^2$ für Methyl oder die Gruppierung

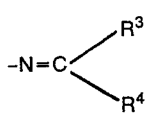

steht, wobei
$R^3$ für Wasserstoff oder Alkyl steht und
$R^4$ für Alkyl, Cycloalkyl, Cycloalkenyl oder gegebenenfalls substituiertes Phenyl steht,
Verfahren zu ihrer Herstellung und ihre Verwendung als Herbizide.



ACTORUM AG

BAYER AKTIENGESELLSCHAFT 5090 Leverkusen, Bayerwerk
Konzernverwaltung RP
Patentabteilung Bi/AB
Ia

# 6-(2,3-Dimethyl-but-2-yl)-1,2,4-triazin-5-on-Derivate

Die vorliegende Erfindung betrifft neue 6-(2,3-Dimethyl-but-2-yl)-1,2,4-triazin-5-on-Derivate, ein Verfahren zu ihrer Herstellung sowie ihre Verwendung als Herbizide, insbesondere als selektive Herbizide.

Es ist bereits bekannt geworden, daß bestimmte substituierte 1,2,4-Triazin-5-on-Derivate, wie beispielsweise 6-Fluor-tert.-butyl-4-isobutylidenamino-3-methylthio-1,2,4-triazin-5-on, als Herbizide verwendet werden können (vergleiche EP-A-0 074 539 [LeA 21 174]). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

Es wurden neue 6-(2,3-Dimethyl-but-2-yl)-1,2,4-triazin-5-on-Derivate der allgemeinen Formel (I)

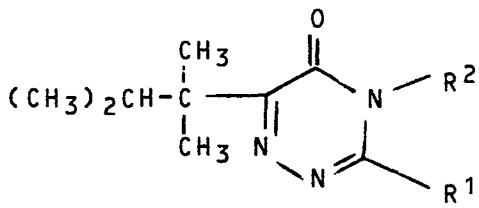

(I)

in welcher

$R^1$ für Alkylthio, Alkylamino oder Dialkylamino steht und

$R^2$ für Methyl oder die Gruppierung 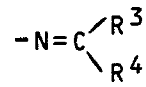 steht, wobei

$R^3$ für Wasserstoff oder Alkyl steht und

$R^4$ für Alkyl, Cycloalkyl, Cycloalkenyl oder gegebenenfalls substituiertes Phenyl steht,

aufgefunden.

Weiterhin wurde gefunden, daß man die 6-(2,3-Dimethyl-but-2-yl)-1,2,4-triazin-5-on-Derivate der Formel (I) erhält, wenn man in einer <u>ersten Stufe</u> 3,3,4-Trimethyl-2-oxo-pentanoylnitril der Formel (II)

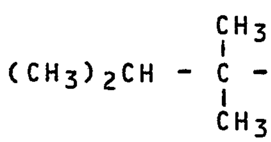

(II)

(a) gegebenenfalls in Gegenwart einer flüssigen Carbonsäure als Lösungsmittel, mit einer anorganischen Säure umsetzt oder

(b) an ein Carboniumion addiert, das aus einem Olefin,

wie z.B. Isobutylen, oder einem tertiären Alkohol, wie z.B. tert.-Butanol, mit einer starken Säure, wie insbesondere Schwefelsäure, gebildet wird, und anschließend der Hydrolyse unterwirft (sog. RITTER-REAKTION)

und

die hierbei entstehenden 3,3,4-Trimethyl-2-oxo-pentancarbonsäureamide der Formel (IIIa)

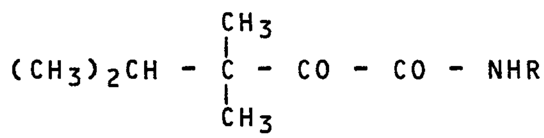

(IIIa)

in welcher

R für Wasserstoff (erhältlich nach Variante a) oder Alkyl, insbesondere tert.-Butyl (erhältlich nach Variante b) steht,

in einer zweiten Stufe in an sich bekannter Weise entweder direkt in der anfallenden Lösung oder nach Zwischenisolierung, gegebenenfalls nach vorheriger Verseifung zur freien 3,3,4-Trimethyl-2-oxo-pentansäure der Formel (III b)

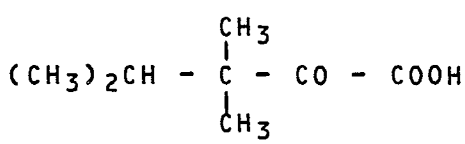

(IIIb)

mit Thiocarbohydraziden der Formel (IV)

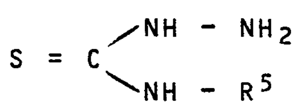

(IV)

in welcher

$R^5$ für Methyl oder Amino steht,

in wässriger bzw. wässrig-saurer Lösung, gegebenenfalls in Gegenwart eines organischen Verdünnungsmittels, zu 6-(2,3-Dimethyl-but-2-yl)-3-mercapto-1,2,4-triazin-5-onen der Formel (V)

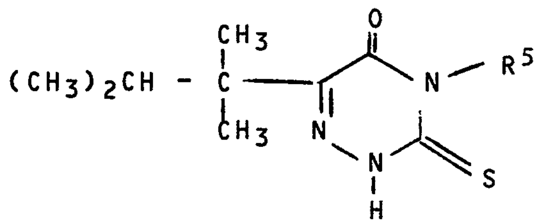

(V)

in welcher

$R^5$ die oben angegebene Bedeutung hat,

umsetzt; diese in einer dritten Stufe in bekannter Weise in alkalischer Lösung mittels Alkylhalogenid, vorzugsweise Alkyliodid oder -bromid, zu 3-Alkylthio-6-(2,3-dimethyl-but-2-yl)-1,2,4-triazin-5-onen der Formeln (Ia) bzw. (VI)

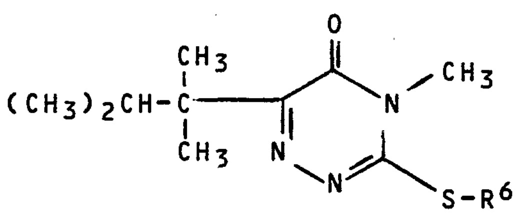

(Ia)

bzw.

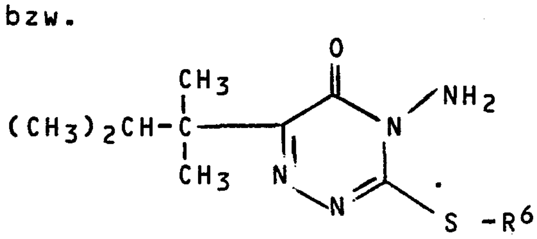

(VI)

in welchen

$R^6$ für Alkyl, vorzugsweise mit 1 bis 4 Kohlenstoffatomen, steht,

alkyliert; diese gegebenenfalls in einer vierten Stufe mit Aminen der Formel (VII)

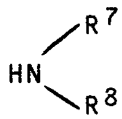

(VII)

in welcher

$R^7$ für Wasserstoff oder Alkyl, vorzugsweise mit 1 bis 6 Kohlenstoffatomen, steht und

$R^8$ für Alkyl, vorzugsweise mit 1 bis 6 Kohlenstoffatomen, steht,

in Gegenwart eines Verdünnungsmittels umsetzt; und gegebenenfalls in einer <u>fünften Stufe</u> die gemäß der dritten bzw. vierten Stufe entstehenden 4-Amino-6-(2,3-dimethyl-but-2-yl)-1,2,4-triazin-5-one der Formel (VIII)

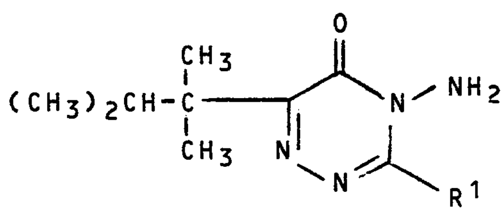

(VIII)

in welcher

$R^1$ die oben angegebene Bedeutung hat,

mit Carbonylverbindungen der Formel (IX)

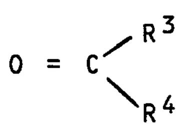

(IX)

in welcher

$R^3$ und $R^4$ die oben angegebene Bedeutung haben,

gegebenenfalls in Gegenwart eines Verdünnungsmittels und gegebenenfalls in Gegenwart eines basischen oder sauren Katalysators umsetzt; die Carbonylverbindungen der

Formel (IX) können dabei gegebenenfalls in Form von Acetalen bzw. Ketalen eingesetzt werden.

Die 6-(2,3-Dimethyl-but-2-yl)-3-mercapto-1,2,4-triazin-5-one der Formel (V) können auch erhalten werden, indem man 1-Chlor-3,3,4-trimethyl-2-pentanon der Formel (X)

$$(CH_3)_2CH - \overset{\displaystyle CH_3}{\underset{\displaystyle CH_3}{\overset{|}{\underset{|}{C}}}} - CO - CH_2Cl \qquad (X)$$

mit Kaliumpermanganat in Gegenwart von Natriumhydroxid zur freien Säure der Formel (IIIb) oxidiert und diese ohne Isolierung gemäß der zweiten Stufe mit Thiocarbohydraziden der Formel (IV) umsetzt.

Außerdem wurde gefunden, daß die 6-(2,3-Dimethyl-2-yl)-1,2,4-triazin-5-on-Derivate der Formel (I) gute herbizide, insbesondere selektiv-herbizide Eigenschaften aufweisen.

Ueberraschenderweise zeigen die erfindungsgemäßen Verbindungen gegenüber dem bekannten 6-Fluor-tert.-butyl-4-isobutylidenamino-3-methylthio-1,2,4-triazin-5-on, welches konstitutionell und wirkungsmäßig eine naheliegende Verbindung ist, neben einer besseren allgemeinen herbiziden Wirkung, insbesondere eine bessere Verträglichkeit bei wichtigen Kulturpflanzen , wie bei Hafer, Weizen, Mais und anderen. Die erfindungsgemäßen Wirkstoffe stellen somit eine Bereicherung der herbiziden Mittel, insbesondere der selektiven chemischen Unkrautbekämpfung dar.

Die erfindungsgemäßen 6-(2,3-Dimethyl-but-2-yl)-1,2,4-triazin-5-on-Derivate sind durch die Formel (I) allgemein definiert. In dieser Formel stehen vorzugsweise

$R^1$ für geradkettiges oder verzweigtes Alkylthio mit 1 bis 4 Kohlenstoffatomen, sowie für Alkylamino und Dialkylamino mit jeweils 1 bis 6 Kohlenstoffatomen in jedem Alkylteil;

$R^2$ für Methyl oder die Gruppierung $-N=CR^3R^4$;

$R^3$ für Wasserstoff oder geradkettiges und verzweigtes Alkyl mit 1 bis 6 Kohlenstoffatomen; und

$R^4$ für geradkettiges oder verzweigtes Alkyl mit 1 bis 6 Kohlenstoffatomen, für Cycloalkyl mit 3 bis 6 Kohlenstoffatomen oder Cycloalkenyl mit 5 oder 6 Kohlenstoffatomen, für gegebenenfalls substituiertes Phenyl, wobei als Substituenten beispielsweise genannt seien: Halogen, Alkyl mit 1 bis 4 Kohlenstoffatomen, Halogenalkyl mit 1 bis 2 Kohlenstoffatomen und bis zu 5 gleichen oder verschiedenen Halogenatomen (wie vorzugsweise Fluor- und Chloratomen), Alkoxy und Alkylthio mit jeweils 1 bis 2 Kohlenstoffatomen, Cyano und Nitro.

Besonders bevorzugt sind diejenigen Verbindungen der Formel (I), in denen

$R^1$ für Methyl-, Ethyl- oder Propylthio; ferner für Methyl-, Ethyl-, Propyl- oder Hexylamino sowie Dimethyl-, Diethyl- oder Ethylmethylamino steht;

$R^2$ für Methyl oder die Gruppierung $-N=CR^3R^4$ steht;

$R^3$ für Wasserstoff oder geradkettiges und verzweigtes Alkyl mit 1 bis 4 Kohlenstoffatomen steht; und

$R^4$ für geradkettiges oder verzweigtes Alkyl mit 1 bis 4 Kohlenstoffatomen, Cyclohexyl, Cyclohexenyl oder für gegebenenfalls substituiertes Phenyl steht, wobei als Substituenten genannt seien:
Fluor, Chlor, Methyl, Ethyl und Nitro.

Verwendet man beispielsweise 3,3,4-Trimethyl-2-oxo-pentanoylnitril als Ausgangsstoff in der ersten Stufe, setzt in der zweiten Stufe die entsprechende freie Säure mit 4-Methylthiosemicarbazid um und verwendet in der dritten Stufe Methyljodid als Alkylierungsmittel, so kann der Reaktionsablauf durch das folgende Formelschema wiedergegeben werden :

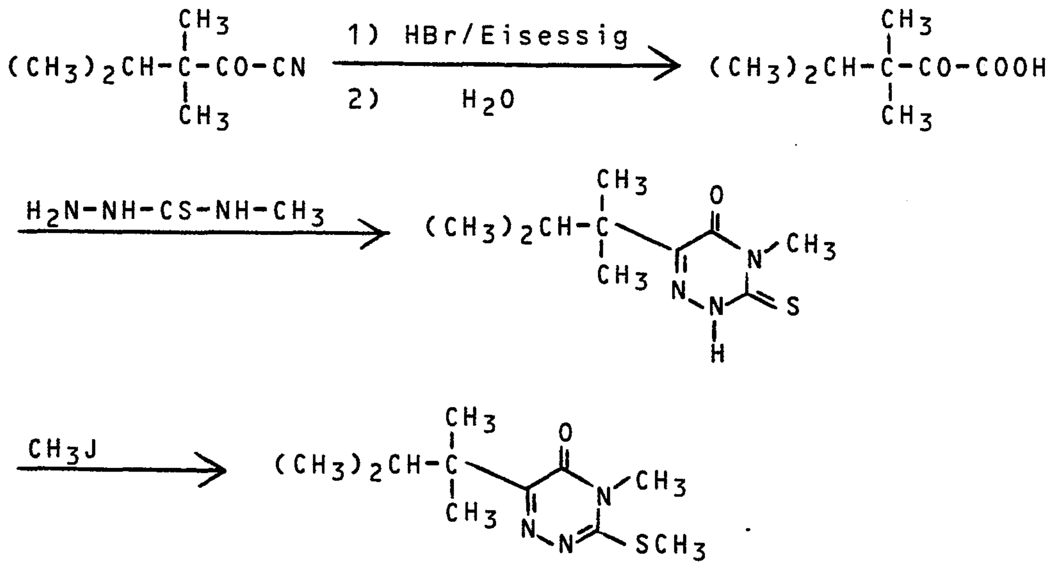


Verwendet man das so erhaltene 6-(2,3-Dimethyl-but-2-yl)-4-methyl-3-methylthio-1,2,4-triazin-5-on und Dimethylamin als Ausgangsstoffe für die vierte Stufe, so kann der Reaktionsablauf durch das folgende Formelschema wiedergegeben werden :

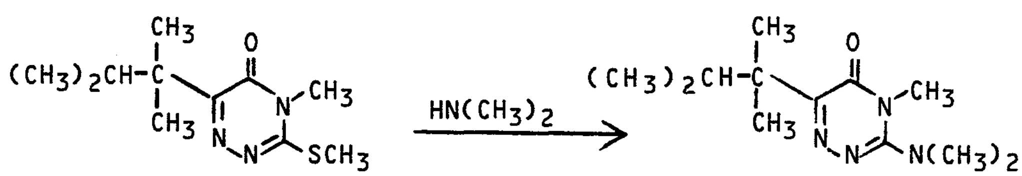

Verwendet man beispielsweise 4-Amino-3-dimethylamino-6-(2,3-dimethyl-but-2-yl)-1,2,4-triazin-5-on und Isobutyraldehyd als Ausgangsstoffe für die fünfte Stufe, so kann der Reaktionsablauf durch das folgende Formelschema wiedergegeben werden :

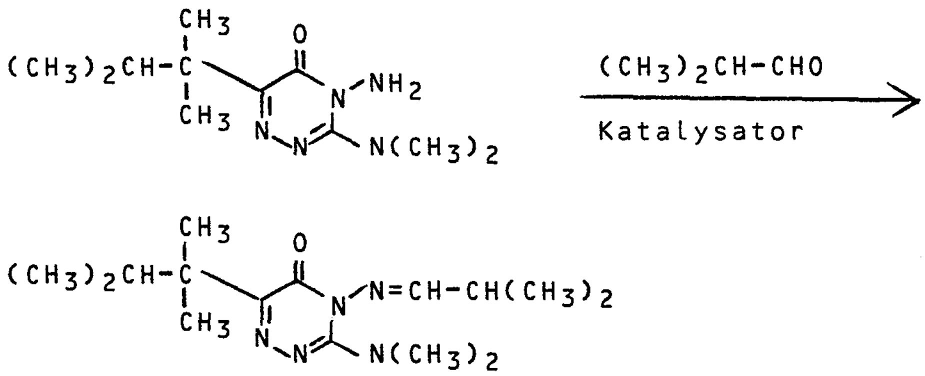


Das für das erfindungsgemäße Verfahren als Ausgangsstoff zu verwendende 3,3,4-Trimethyl-2-oxo-pentanoylnitril der Formel (II) ist Gegenstand einer eigenen älteren Anmeldung, die noch nicht veröffentlicht ist (vgl. die Deutsche Patentanmeldung P 32 40 308 vom 30.10.1982 [LeA 21 937]). Es läßt sich in prinzipiell bekannter Weise aus dem Chlorketon der Formel (X)

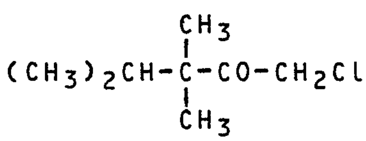

(X)

herstellen, indem man dieses zunächst mit Natriumhydroxid und Brom in Gegenwart eines Verdünnungsmittels, wie beispielsweise Dioxan, bei Temperaturen zwischen

0°C und 10°C in die Carbonsäure der Formel (XI)

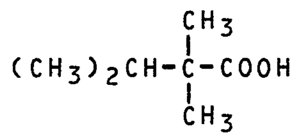 (XI)

überführt, diese anschließend mit Thionylchlorid in Gegenwart eines Verdünnungsmittels, wie beispielsweise Chloroform, bei Temperaturen zwischen 15°C und 30°C in das entsprechende Säurechlorid überführt, welches schließlich mit Trimethylsilylcyanid bei Temperaturen zwischen 110°C und 130°C umgesetzt wird.

Das Chlorketon der Formel (X) ist ebenfalls Gegenstand einer eigenen älteren Anmeldung, die noch nicht veröffentlich ist (vgl. die Deutsche Patentanmeldung P 32 24 129 vom 29.6.1982 [LeA 21 717]). Man erhält es aus 2,3-Dimethyl-1-buten, indem man zunächst mit Chlorwasserstoff bei 10°C bis 20°C 2-Chlor-2,3-dimethylbutan herstellt, an dieses in Gegenwart eines Katalysators, wie z.B. Aluminiumtrichlorid, bei Temperaturen von 0°C bis 10°C 1,1-Dichlorethylen anlagert, wobei man den ungesättigten Chlorkohlenwasserstoff der Formel (XII)

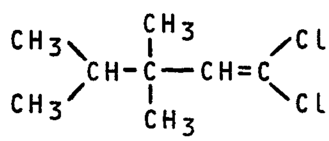 (XII)

erhält. Daraus stellt man mit Natriumphenolat in Gegenwart eines basischen Lösungsmittels, wie z.B. N-Methylpyrrolidon, bei Temperaturen zwischen 195°C und 220°C

den Enolether der Formel (XIII) her,

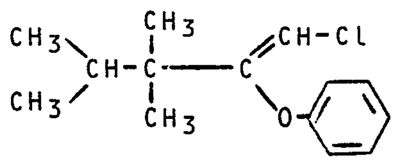

(XIII)

der sich in Gegenwart einer Säure, wie z.B. Ameisensäure, bei 80°C bis 100°C zum gewünschten Chlorketon der Formel (X) hydrolysieren läßt. Die Ausgangsverbindung 2,3-Dimethyl-1-buten ist bekannt (vgl. Beilstein Band 1, S. 218).

Die Thiocarbohydrazide der Formel (IV), die Amine der Formel (VII) und die Carbonylverbindungen der Formel (IX) sind allgemein bekannte Verbindungen der organischen Chemie.

Die erste Stufe des erfindungsgemäßen Verfahrens kann in Abwesenheit oder in Gegenwart einer unter den Reaktionsbedingungen flüssigen, niederaliphatischen Carbonsäure als Lösungsmittel durchgeführt werden. Als solche Lösungsmittel kommen vorzugsweise Carbonsäuren mit 1 bis 6 Kohlenstoffatomen, wie Essigsäure, Propionsäure oder Ameisensäure, infrage.

Die erste Stufe des erfindungsgemäßen Verfahrens wird mit Hilfe einer starken anorganischen Säure durchgeführt. Hierzu gehören vorzugsweise die Halogenwasserstoffsäuren, wie Chlorwasserstoff und Bromwasserstoff, sowie konzentrierte Schwefelsäure.

Die Reaktionstemperaturen können bei der Durchführung dieser Verfahrensstufe in einem größeren Bereich variiert werden. Im allgemeinen arbeitet man zwischen -20°C und +50°C, vorzugsweise zwischen 0°C und +40°C.

Bei der Durchführung der ersten Stufe des erfindungsgemäßen Verfahrens setzt man auf 1 Mol Nitril der Formel (II) vorzugsweise 1 bis 10 Mol an anorganischer Säure ein.

Die zweite Stufe des erfindungsgemäßen Verfahrens erfolgt vorzugsweise ohne vorherige Zwischenisolierung der 3,3,4-Trimethyl-2-oxo-pentancarbonsäureamide der Formel (IIIa) und nach Verseifung derselben in üblicher Weise zu freien Säuren der Formel (IIIb).

Die zweite Stufe des erfindungsgemäßen Verfahrens wird in wäßriger Lösung bzw. in Gegenwart einer wäßrig-sauren Lösung wie einer halogenwassserstoffsauren, vorzugsweise einer salzsauren oder einer schwefelsauren Lösung durchgeführt.

Wird in Gegenwart eines organischen Lösungsmittels gearbeitet, so kommen alle üblichen organischen Lösungsmittel in Frage, wie insbesondere Dimethylformamid.

Die Reaktionstemperaturen können bei der Durchführung der zweiten Stufe des erfindungsgemäßen Verfahrens in einem größeren Bereich variiiert werden. Im allgemeinen arbeitet man zwischen 0 und 120°C, vorzugsweise zwischen 0 und 100°C.

Bei der Durchführung der zweiten Stufe des erfindungsgemäßen Verfahrens setzt man die Ausgangsstoffe vorzugsweise in molaren Mengen bzw. mit einem Überschuß an Thiocarbohydrazid der Formel (IV) ein. Die Isolierung der Zwischenprodukte der Formel (V) erfolgt in üblicher Weise.

Die dritte Stufe des erfindungsgemäßen Verfahrens wird in Gegenwart einer Base durchgeführt. Hierbei werden vorzugsweise Alkalihydroxide, wie Natriumhydroxid, in wäßriger Lösung oder Alkalialkoholate, wie Natriummethylat, wobei überschüssiger Alkohol als Lösungsmittel verwendet wird, eingesetzt.

Die Reaktionstemperaturen können bei der Durchführung der dritten Stufe des erfindungsgemäßen Verfahrens in einem größeren Bereich variiert werden. Im allgemeinen arbeitet man zwischen 0 und 100°C, vorzugsweise zwischen 0 und 50°C.
Bei der Durchführung der dritten Stufe des erfindungsgemäßen Verfahrens setzt man auf 1 Mol Zwischenprodukt der Formel (V) vorzugsweise 1 bis 1,5 Mol Alkylierungsmittel ein. Die Isolierung der Zwischenprodukte bzw. Endprodukte der Formel (Ia) erfolgt in üblicher Weise.

Als Verdünnungsmittel kommen für die vierte Stufe des erfindungsgemäßen Verfahrens vorzugsweise organische Lösungsmittel in Frage, wie insbesondere Isopropanol/Eisessig.

Die Reaktionstemperaturen können bei der Durchführung der vierten Stufe des erfindungsgemäßen Verfahrens in einem größeren Bereich variiert werden. Im allgemeinen arbeitet man zwischen 20 und 180°C, vorzugsweise zwischen 40 und 150°C.

Bei der Durchführung der vierten Stufe des erfindungsgemäßen Verfahrens setzt man auf 1 Mol der Verbindungen der Formel (Ia) bzw. der Formel (VI) vorzugsweise 1 bis 3 Mol Amin der Formel (VII) ein. Die Isolierung der Endprodukte erfolgt in üblicher Weise.

Als Verdünnungsmittel kommen für die fünfte Stufe des erfindungsgemäßen Verfahrens alle inerten organischen Lösungsmittel in Frage. Hierzu gehören vorzugsweise Alkohole, wie Methanol, Ethanol und tert.-Butanol; Ether, wie insbesondere Tetrahydrofuran und Dioxan; halogenierte Kohlenwasserstoffe, wie Methylenchlorid; sowie aromatische Kohlenwasserstoffe, wie Benzol, Toluol oder Xylol.

Die fünfte Stufe des erfindungsgemäßen Verfahrens wird gegebenenfalls in Gegenwart eines basischen oder eines sauren Katalysators durchgeführt. Hierbei können alle üblichen basischen Katalysatoren eingesetzt werden, wie z.B. Dimethylbenzylamin. Als saure Katalysatoren kommen insbesondere p-Toluolsulfonsäure oder saure Kationenaustauscher in Betracht.

Die Reaktionstemperaturen können bei der Durchführung der fünften Stufe des erfindungsgemäßen Verfahrens in einem größeren Bereich variiert werden. Im allgemeinen arbeitet man bei Temperaturen zwischen -50 und +150°C, vorzugsweise zwischen -10 und + 120°C.

Bei der Durchführung der fünften Stufe des erfindungsgemäßen Verfahrens setzt man vorzugsweise auf 1 Mol 4-Amino-6-(2,3-dimethyl-but-2-yl)-1,2,4-triazin-5-on der Formel (VIII) 1 bis 2 Mol einer Carbonylverbindung der Formel (IX) und gegebenenfalls 0,01 bis 0,1 Mol Katalysator ein. Die Isolierung der Verbindungen der Formel (I) erfolgt in üblicher Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additve können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide, wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,05 und 10 kg Wirkstoff pro Hektar Bodenfläche, vorzugsweise zwischen 0,1 und 5 kg pro ha.

Die nachfolgenden Beispiele dienen zur weiteren Erläuterung der Erfindung.

Herstellungsbeispiele

Beispiel 1 :

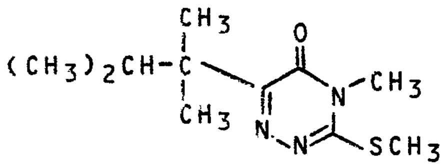

(1)

(1. und 2. Stufe)

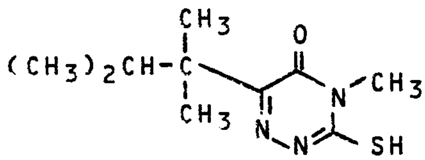

In 98,0 g (1,0 Mol) konzentrierte Schwefelsäure werden bei Raumtemperatur 51,2 g (0,5 Mol) Essigsäureanhydrid und 34,8 g (0,025 Mol) 3,3,4-Trimethyl-2-oxo-pentanoylnitril eingetragen. Nach einstündigem Nachrühren bei 40°C wird das Reaktionsgemisch in eine Lösung von 23,3 g (0,25 Mol) 4-Methyl-thiosemicarbazid in 2 ml 1 n Salzsäure eingerührt. Nach beendeter Zugabe läßt man eine Stunde nachrühren. Anschließend wird mit 1200 ml 2n Natronlauge ein pH-Wert von ca. 5 eingestellt und eine Stunde auf 80°C erwärmt. Nach dem Abkühlen wird das ausgefallene Reaktionsprodukt abgesaugt, mit 200 ml Wasser gewaschen und getrocknet.

Man erhält 36,0 g (63 % der Theorie) 6-(2,3-Dimethylbut-2-yl)-3-mercapto-4-methyl-1,2,4-triazin-5-on vom Schmelzpunkt 196°C.

0130519

(3. Stufe)

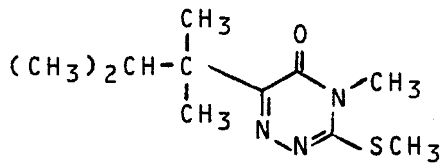

(1)

6,0 g (0,026 Mol) 6-(2,3-Dimethyl-but-2-yl)-3-mercapto-4-methyl-1,2,4-triazin-5-on (gemäß Stufe 2) werden in 29 ml 1n Natronlauge gelöst. Nach vollständiger Lösung des Produktes werden 4,1 g Methyljodid zugetropft. Man läßt bei Raumtemperatur über Nacht rühren. Danach wird der entstandene Feststoff abgesaugt, mit Wasser gewaschen und getrocknet.

Man erhält 4,7 g (75 % der Theorie) 6-(2,3-Dimethyl-but-2-yl)-4-methyl-3-methylthio-1,2,4-triazin-5-on vom Schmelzpunkt 103°C bis 104°C.

Herstellung der Ausgangsprodukte

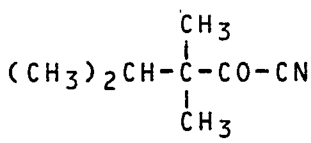

266 g (1,79 Mol) 2,2,3-Trimethylbutanoylchlorid werden auf 120°C erwärmt und dann tropfenweise mit 177 g (1,79 Mol) Trimethylsilylcyanid versetzt, wobei die Mischung refluxiert. Während 16-stündigem Erhitzen destilliert freigesetztes Trimethylsilylchlorid langsam über einen Anschütz-Aufsatz ab und wird in einer Vorlage aufgefangen. Danach wird das Reaktionsgemisch im Wasserstrahlvakuum über eine Spiegelkolonne destilliert. Man erhält 213,2 g (89 % der Theorie) an 3,3,4-Trimethyl-2-oxo-pentanoylnitril vom Siedepunkt 55°C bis 56°C/24 mbar.

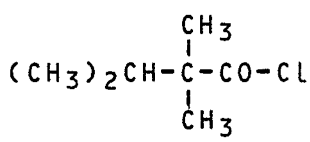

252 g (1,95 Mol) 2,2,3-Trimethylbuttersäure in 150 ml absolutem Chloroform werden bei Raumtemperatur tropfenweise unter Rühren mit 219 ml (2,96 Mol) Thionylchlorid versetzt und nach beendeter Zugabe weitere 5 Stunden bei Raumtmperatur gerührt. Zur Aufarbeitung destilliert man das Lösungsmittel ab und fraktioniert den Rückstand im Wasserstrahlvakuum. Man erhält 266,6 g (92,2 % der Theorie) an 2,2,3-Trimethylbutanolylchlorid vom Siedepunkt 45°C bis 48°C/14 bis 18 mbar.

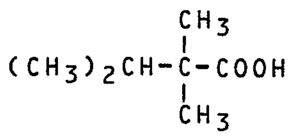

200 g (1,23 Mol) 1-Chlor-3,3,4-trimethyl-2-petanon in 900 ml Dioxan werden unter Rühren und Eiskühlung bei 0°C bis 5°C zu einer Lösung von 492 g (12,3 Mol) Natriumhydroxid und 125,2 g (2,44 Mol) Brom in 2.200 ml Wasser getropft. Man rührt die Mischung 16 Stunden bei Raumtemperatur. Vor der Aufarbeitung zerstört man überschüssiges Natriumhypobromit durch Zugabe von 122 g (0,64 Mol) Natriumpyrosulfit ($Na_2S_2O_5$). Nach 15 minütigem Rühren ist kein überschüssiges Hypobromit mehr nachweisbar. Die wässrige Dioxanphase wird abgetrennt, mit konzentrierter Salzsäure angesäuert und mit Essigester mehrfach extrahiert. Man wäscht die vereinigten organischen Phasen mit Wasser, trocknet über Natriumsulfat und entfernt das Lösungsmittel im Vakuum. Man erhält 126,8 g (79,2 % der Theorie) an 2,2,3-Trimethylbuttersäure als Oel.

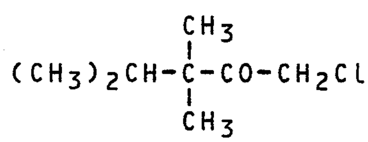

238 g (1 Mol) 1-Chlor-2-phenoxy-3,3,4-trimethyl-1-penten werden mit 500 g Ameisensäure und 50 g konzentrierter Salzsäure 2 Stunden bei 80°C gerührt. Man verdünnt mit Methylenchlorid, wäscht einmal mit Wasser und viermal mit verdünnter Natronlauge. Nach dem Trocknen über Natriumsulfat wird das Lösungsmittel im Vakuum abgezogen und der Rückstand über eine Kolonne destilliert. Man er-

hält 125 g bis 135 g (77 % bis 83 % der Theorie) an 1-Chlor-3,3,4-trimethyl-2-pentanon vom Siedepunkt 88°C bis 93°C/16 mbar.

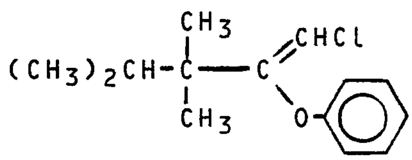

In 1,6 l ca. 100°C heißes N-Methylpyrrolidon werden 487 g Natriumphenolat eingetragen und durch Erhitzen auf 200°C gelöst. Im Verlauf von 3 Stunden werden 360 g (2 Mol) 1,1-Dichlor-3,3,4-trimethyl-1-penten so zugetropft, daß die Temperatur der Reaktionsmischung nicht unter 195°C sinkt. Anschließend erhitzt man 8 Stunden auf 200°C bis 210°C. Zur Aufarbeitung wird die Lösung mit Methylenchlorid verdünnt und mit verdünnter Natronlauge ausgeschüttelt. Nach Trocknen und Abziehen des Lösungsmittels bleiben 406 g Rohprodukt zurück, das bei 105°C bis 120°C/0,1 mbar destilliert wird. Man erhält 337 g (71 % der Theorie) an 1-Chlor-2-phenoxy-3,3,4-trimethyl-1-penten.

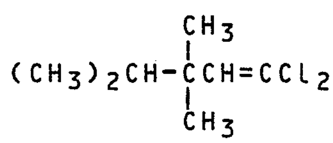

In einer trockenen Apparatur mit Trockenrohr gibt man 20 g wasserfreies, gepulvertes Aluminiumchlorid portionsweise zu 2.040 g (21 Mol) Vinylidenchlorid bei -10°C. Dann tropft man im Temperaturbereich von 0°C bis 10°C 837 g (7 Mol) 2-Chlor-2,3-dimethylbutan zu. Man läßt auf 20°C erwärmen und gibt weitere Aluminiumchlorid-Portionen (maximal 20 g) unter Kühlung zu, bis

die Reaktion nicht mehr exotherm ist. Der Ansatz wird 4 Stunden bei Raumtemperatur nachgerührt, durch Eintropfen von 70 ml Essigsäure wird der Katalysator desaktiviert und die Mischung wird über Natriumsulfat filtriert. Durch Eintropfdestillation bei 1 mbar werden die flüchtigen Bestandteile von den schwerflüchtigen getrennt. Das Destillat wird an einer Kolonne fraktioniert. Man erhält 824 g bis 1.226 g (65 % bis 95 % der Theorie) an 1,1-Dichlor-3,3,4-trimethyl-1-penten vom Siedepunkt 60°C bis 75°C/20 mbar.

$$(CH_3)_2CH-\overset{\overset{\displaystyle CH_3}{|}}{\underset{\underset{\displaystyle CH_3}{|}}{C}}-Cl$$

In 840 g (10 Mol) 2,3-Dimethyl-1-buten werden bei 10°C bis 20°C unter Eiskühlung ca. 440 g (12 Mol) Chlorwasserstoff aus der Bombe in 16 Stunden eingeleitet. Laut IR ist vollständiger Umsatz erfolgt. Ueberschüssiger Chlorwasserstoff wird in die Wasserstrahlpumpe gezogen. Man erhält 1.103 g (91 % der Theorie) an 2-Chlor-2,3-dimethyl-butan.

0130519

Beispiel 2

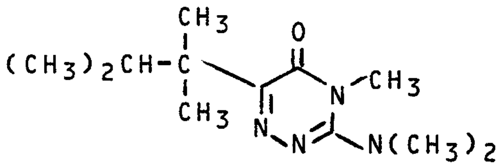

(2)

In eine Lösung von 6,0 g (0,1 Mol) Eisessig in 150 ml Isopropanol wird bei 0°C bis 5°C ca. 0,1 Mol Dimethylamin eingeleitet. Diese Reaktionslösung wird anschließend mit 5,6 g (0,0232 Mol) 6-(2,3-Dimethyl-but-2-yl)-4-methyl-3-methylthio-1,2,4-triazin-5-on (Beispiel 1) versetzt. Man läßt auf Raumtemperatur erwärmen und erhitzt anschließend 16 Stunden unter Rückfluß. Danach wird das Reaktionsgemisch eingeengt und der ölige Rückstand in Wasser/Methylenchlorid eingerührt und zweimal mit 1n Salzsäure extrahiert. Die vereinigten Salzsäurephasen werden mit konzentrierter Natronlauge unter Eiskühlung auf pH 14 gebracht und mehrfach mit Methylenchlorid extrahiert. Die vereinigten organischen Phasen werden mit Wasser gewaschen, über Natriumsulfat getrocknet und im Vakuum vom Lösungsmittel befreit. Der ölige Rückstand wird in Petrolether verrieben. Man erhält 4,1 g (74,1 % der Theorie) an 3-Dimethylamino-6-(2,3-dimethyl-but-2-yl)-4-methyl-1,2,4-triazin-5-on vom Schmelzpunkt 88°C bis 89°C.

Beispiel 3

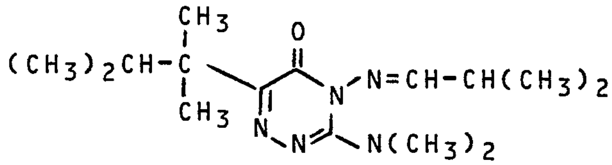

(3)

Zu 12,0 g (0,05 Mol) 4-Amino-3-dimethylamino-6-(2,3-dimethyl-but-2-yl)-1,2,4-triazin-5-on und 0,5 g p-Toluolsulfonsäure in 150 ml Toluol werden 4,32 g (0,06 Mol) Isobutyraldehyd gegeben. Die Reaktionslösung wird über Nacht unter Rückfluß am Wasserabscheider erhitzt. Danach läßt man abkühlen, wäscht mit 1n Natronlauge und Wasser, trocknet über Natriumsulfat und engt ein. Nach Trennung über eine Kieselgelsäule (Kieselgel 60; Laufmittel Ether/Petrolether 3 : 1) erhält man 3,2 g (22 % der Theorie) an 3-Dimethylamino-6-(2,3-dimethyl-but-2-yl)-4-isobutylidenamino-1,2,4-triazin-5-on vom Brechungsindex $n_D^{20}$ = 1,531.

Herstellung des Ausgangsproduktes

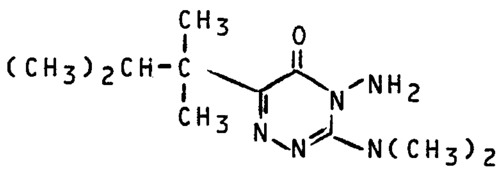

Zu 5,8 g (0,097 Mol) Eisessig in 100 ml Isopropanol leitet man bei 10°C 4,5 g (0,1 Mol) Dimethylamin ein. Zu dieser Lösung gibt man 11,7 g (0,048 Mol) 4-Amino-6-(2,3-dimethyl-2-butyl)-3-methylthio-1,2,4-triazin-5-on und erhitzt langsam bis zum Rückfluß. Man hält die Rückflußtemperatur unter Rühren über 15 Stunden aufrecht, läßt das Reaktionsgemisch abkühlen und entfernt das Lösungsmittel im Vakuum. Der Rückstand wird in Dichlormethan aufgenommen und zweimal mit 1n Salzsäure extrahiert. Die vereinigten Salzsäurephasen werden mit konzentrierter Natronlauge unter Eiskühlung auf pH 14 gebracht und mehrfach mit Dichlormethan extrahiert. Die vereinigten organischen Phasen werden mit Wasser gewaschen, über Natriumsulfat getrocknet und im Vakuum vom Lösungsmittel befreit. Das zurückbleibende Oel kristallisiert beim Verreiben mit Petrolether. Man erhält 7,3 g (63 % der Theorie) an 4-Amino-3-dimethylamino-6-(2,3-dimethyl-but-2-yl)-1,2,4-triazin-5-on vom Schmelzpunkt 84°C bis 86°C.

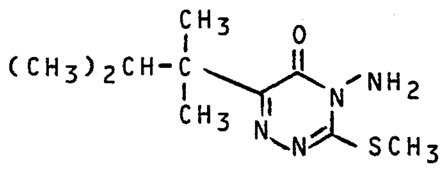

15,0 g (0,0697 Mol) 4-Amino-6-(2,3-dimethyl-2-butyl)-3-mercapto-1,2,4-triazin-5-on werden in 77 ml 1n Natronlauge gelöst und vom unlöslichen Rückstand abfiltriert. Das Filtrat wird bei Raumtemperatur mit 10,9 g (0,77 Mol) Methyljodid versetzt und 12 bis 15 Stunden gerührt. Zur Aufarbeitung saugt man den ausgefallenen Feststoff ab und wäscht ihn mehrfach mit Wasser. Die Trocknung erfolgt bei 40°C bis 50°C im Vakuum bis zur Gewichtskonstanz. Man erhält 11,7 g (69,3 % der Theorie) an 4-Amino-6-(2,3-dimethyl-2-butyl)-3-methylthio-1,2,4-triazin-5-on vom Schmelzpunkt 127°C bis 128°C.

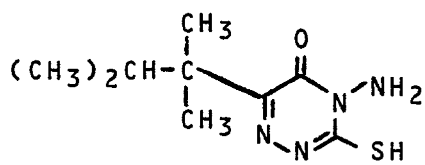

49 g (0,353 Mol) 3,3,4-Trimethyl-2-oxo-pentanoylnitril werden in 283 g einer 33 %-igen Bromwasserstofflösung in Eisessig 4,5 Stunden bei 15°C bis 20°C gerührt. Danach fügt man tropfenweise unter Rühren und Eiskühlung eine Lösung von 41,2 g (0,388 Mol) Thiocarbohydrazid in 388 ml 1n Salzsäure zu, so daß die Temperatur der Reaktionsmischung 20°C nicht übersteigt. Nach beendeter Zugabe rührt man weitere 3 Tage bei Raumtemperatur. Danach wird das ausgefallene kristalline Produkt abgesaugt, mit Petrolether gewaschen und getrocknet. Man erhält 26,3 g (35 % der Theorie) an 4-Amino-6-(2,3-dimethyl-2-butyl)-3-mercapto-1,2,4-triazin-5-on vom Schmelzpunkt 266°C bis 270°C.

In analoger Weise und entsprechend dem erfindungsgemäßen Verfahren werden die in der nachfolgenden Tabelle aufgeführten Verbindungen der Formel (I)

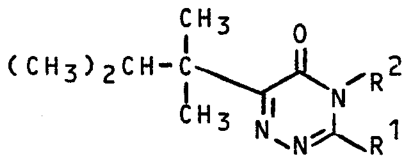

(I)

erhalten :

Tabelle 1

| Beisp.-Nr. | $R^1$ | $R^2$ | Schmelzpunkt (°C) |
|---|---|---|---|
| 4 | $-N(CH_3)_2$ | $-N=CH-$ 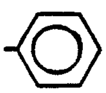 | 95 |

Verwendungsbeispiel

In dem nachfolgenden Verwendungbeispiel wird die nachstehend angegebene Verbindung als Vergleichssubstanz eingesetzt :

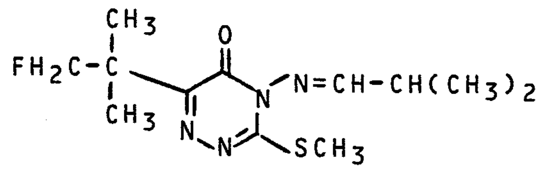 (A)

6-Fluor-tert.-butyl-4-isobutylidenamino-3-methylthio-1,2,4-triazin-5-on (bekannt aus EP-A-0 074 539/ DE-OS 31 35 413).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die Verbindung gemäß Herstellungsbeispiel (3) neben einer besseren allgemeinen herbiziden Wirksamkeit insbesondere eine bessere Selektivität in Hafer, Weizen und Mais als die aus dem Stand der Technik bekannte Verbindung (A).

Patentansprüche

1. 6-(2,3-Dimethyl-but-2-yl)-1,2,4-triazin-5-on-Derivate der allgemeinen Formel (I)

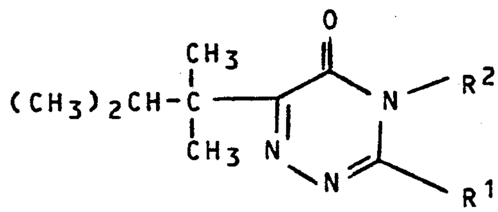

(I)

in welcher

$R^1$ für Alkylthio, Alkylamino oder Dialkylamino steht und

$R^2$ für Methyl oder die Gruppierung 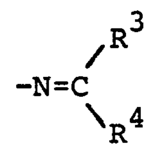 steht, wobei

$R^3$ für Wasserstoff oder Alkyl steht und

$R^4$ für Alkyl, Cycloalkyl, Cycloalkenyl oder gegebenenfalls substituiertes Phenyl steht.

2. 3-Dimethylamino-6-(2,3-dimethyl-but-2-yl)-4-isobutylidenamino-1,2,4-triazin-5-on der Formel

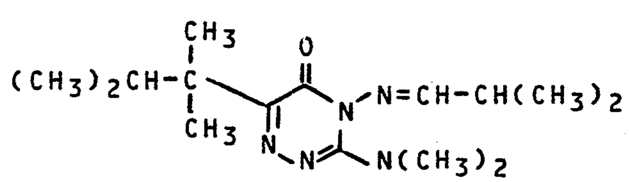

(3)

gemäß Anspruch 1.

3. Verfahren zur Herstellung von 6-(2,3-Dimethyl-but-2-yl)-1,2,4-triazin-5-on-Derivaten der allgemeinen Formel (I) gemäß Anspruch 1, dadurch gekennzeichnet, daß man in einer <u>ersten Stufe</u> 3,3,4-Trimethyl-2-oxo-pentanoylnitril der Formel (II)

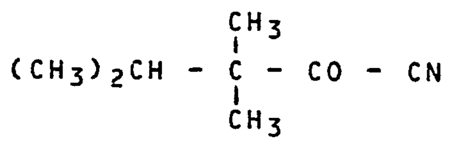

(II)

(a) gegebenenfalls in Gegenwart einer flüssigen Carbonsäure als Lösungsmittel, mit einer anorganischen Säure umsetzt oder

(b) an ein Carboniumion addiert, das aus einem Olefin oder einem tertiären Alkohol mit einer starken Säure gebildet wird, und anschließend der Hydrolyse unterwirft (sog. RITTERReaktion)

und

die hierbei entstehenden 3,3,4-Trimethyl-2-oxo-pentancarbonsäureamide der Formel (IIIa)

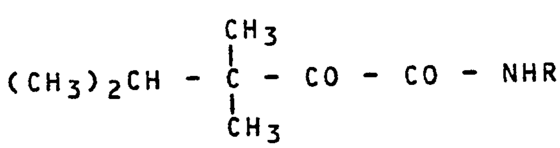

(IIIa)

in welcher

R für Wasserstoff (erhältlich nach Variante a) oder Alkyl, insbesondere tert.-Butyl (erhältlich nach Variante b) steht, in einer <u>zweiten</u> Stufe in an sich bekannter Weise entweder direkt in der anfallenden Lösung oder nach Zwischenisolierung, gegebenenfalls nach vorheriger Verseifung zur freien 3,3,4-Trimethyl-2-oxo-pentansäure der Formel (IIIb)

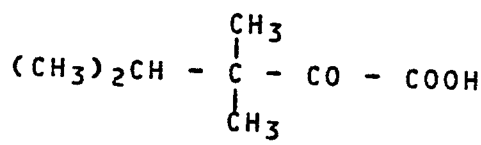

(IIIb)

mit Thiocarbohydraziden der Formel (IV)

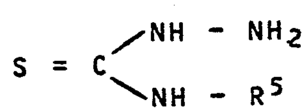

(IV)

in welcher

$R^5$ für Methyl oder Amino steht,

in wäßriger bzw. wäßrig-saurer Lösung, gegebenenfalls in Gegenwart eines organischen Verdünnungsmittels, zu 6-(2,3-Dimethyl-but-2-yl)-3-mercapto-1,2,4-triazin-5-onen der Formel (V)

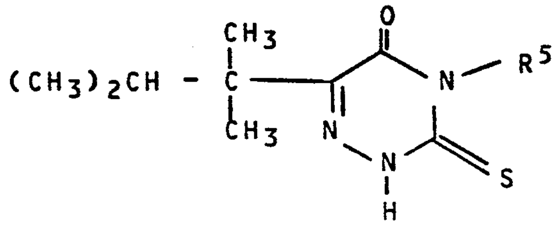

(V)

in welcher

$R^5$ die oben angegebene Bedeutung hat,

umsetzt; diese in einer <u>dritten Stufe</u> in bekannter Weise in alkalischer Lösung mittels Alkylhalogenid, vorzugsweise Alkyliodid oder -bromid, zu 3-Alkylthio-6-(2,3-dimethyl-but-2-yl)-1,2,4-triazin-5-onen der Formeln (Ia) bzw. (VI)

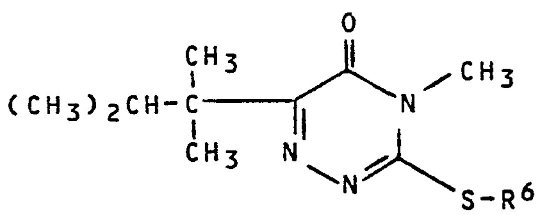

(Ia)

bzw.

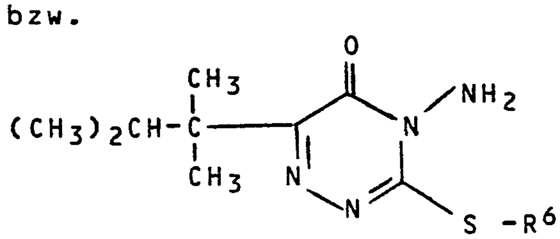

(VI)

in welchen

$R^6$ für Alkyl, vorzugsweise mit 1 bis 4 Kohlenstoffatomen, steht,

alkyliert; diese gegebenenfalls in einer <u>vierten Stufe</u> mit Aminen der Formel (VII)

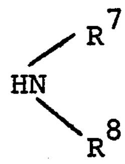

(VII)

in welcher

$R^7$ für Wasserstoff oder Alkyl, vorzugsweise mit 1 bis 6 Kohlenstoffatomen, steht und

$R^8$ für Alkyl, vorzugsweise mit 1 bis 6 Kohlenstoffatomen steht,

in Gegenwart eines Verdünnungsmittels umsetzt; und gegebenenfalls in einer <u>fünften Stufe</u> die gemäß der dritten bzw. vierten Stufe entstehenden 4-Amino-6-(2,4-dimethyl-but-2-yl)-1,2,4-triazin-5-one der Formel (VIII)

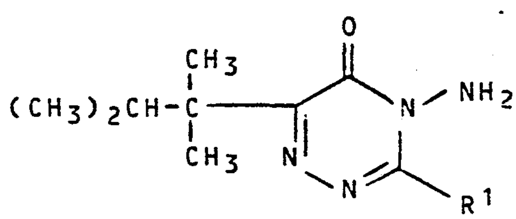

(VIII)

in welcher

$R^1$ die oben angegebene Bedeutung hat,

mit Carbonylverbindungen der Formel (IX)

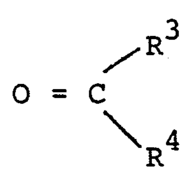

(IX)

in welcher

$R^3$ und $R^4$ die oben angegebene Bedeutung haben,

gegebenenfalls in Gegenwart eines Verdünnungsmittels und gegebenenfalls in Gegenwart eines basischen oder sauren Katalysators umsetzt, wobei die Carbonylverbindungen der Formel (IX) gegebenenfalls in Form von Acetalen bzw. Ketalen eingesetzt werden können.

4. Herbizide Mittel, gekennzeichnet durch einen Gehalt an mindestens einem 6-(2,3-Dimethyl-but-2-yl)-1,2,4-triazin-5-on-Derivat der Formel (I) gemäß Anspruch 1.

5. Verwendung von 6-(2,3-Dimethyl-but-2-yl)-1,2,4-triazin-5-on-Derivaten der Formel (I) gemäß Anspruch 1 zur Bekämpfung von unerwünschtem Pflanzenwachstum.

6. Verfahren zur Herstellung von herbiziden Mitteln, dadurch gekennzeichnet, daß man 6-(2,3-Dimethyl-but-1-yl)-1,2,4-triazin-5-on-Derivate der Formel (I) gemäß Anspruch 1 mit Streckmitteln und/oder oberflächenaktiven Mitteln vermischt.